# EUROPEAN PATENT SPECIFICATION

(11) **EP 1 032 434 B1**
(45) Date of publication and mention of the grant of the patent: **12.03.2003**
(21) Application number: 98948051.2
(22) Date of filing: 08.10.1998
(51) Int. Cl.: A61L 15/36, A61F 13/15

(54) **PRODUCT AND PREPARATION CONTAINING LACTIC ACID BACTERIA**
MILCHSÄUREBAKTERIEN ENTHALTENDES PRODUKT UND HERSTELLUNG
PRODUIT ET PREPARATION RENFERMANT DES BACTERIES LACTIQUES

(30) Priority: 08.10.1997 SE 9703669
(43) Date of publication of application: 06.09.2000
(73) Proprietor: SCA Hygiene Products AB, 405 03 Göteborg (SE)
(72) Inventor: ANDERSSON, Rolf, S-435 31 Mölnlycke (SE); RUNEMAN, Bo, S-433 75 Jonsered (SE); FORSGREN-BRUSK, Ulla, S-435 43 Mölnlycke (SE); GRAHN HAKANSSON, Eva, S-903 31 Ume (SE); HAKANSSON, Stellan, S-903 31 Ume (SE); HOLM, Stig, E., S-907 38 Ume (SE)
(74) Representative: Bergstrand, Mikael Gudmundsson
(86) International application number: SE9801816
(87) International publication number: WO99017813

(56) References cited:
- WO-A1-92/13577
- WO-A1-93/09793
- WO-A1-97/02846

## Description

### FIELD OF INVENTION

The present invention relates to an absorbent article that contains lactic acid bacteria and that is intended to be brought into contact with a user's skin in the perineum.

### BACKGROUND OF THE INVENTION

Infections in the urogenital region is a problem that affects many individuals. In and around the anus, there are many different kinds of microorganisms as well as a large amount of these microorganisms. It is known that one reason for many infections in the urogenital region is that microorganisms from a person's own intestinal flora spread from the anus to urogenital organs over perineum and there cause infection.

Normally, an ecological balance prevails between different microorganisms on skin and mucus membrane, and the normal microbiological flora is highly significant in preventing the establishment of undesirable microorganisms. Lactic acid bacteria, *inter alia*, play an active role in this respect. However, situations are found where this natural defence system is inadequate and is disturbed in a way that enables potentially pathogenic microorganisms to become established and give rise to infection, for instance in conjunction with medication, poor hygiene, skin changes and changes in mucus membranes.

Microorganisms that can be associated with the occurrence of these problems are, e.g., microorganisms from the genera Escherichia, Enterococcus, Proteus, Klebsiella, Streptococcus, Gardnerella and Candida.

With regard to the danger of contracting infections in the urogenital region, old and young women are more at risk than men, due to the short distance from the anus to the urethra orifice and vagina.

Groups that are even more at risk in this respect are young girls who do not yet have a developed flora of lactic acid bacteria in the urogenital region, and older women who no longer have a developed flora of this nature.

Another risk group are individuals who have been treated with antibiotics against some other type of infection, resulting in a change in their general, natural microbiological flora and then also in the urogenital region.

A closely related problem resides in vaginal colonisation from the anus of, e.g., streptococci, etc. A large percentage of adult women (∼30%) carry group B streptococci vaginally. Pregnant women from this group are a particular risk group, since the foetus or the newly born child can be contaminated and develop a serious infection.

A closely related problem is bacterial vaginosis. A large proportion of adult women (∼10%) suffer from this. Pregnant women that have such problems constitute a risk group, since the condition can lead to a premature birth which constitutes a serious risk to the child's health.

Recurrent urinary tract infections are general in the case of many individuals, and the occurrence of such infections can lead to complications in the form of kidney damage, for instance, when relevant treatment is not available.

A natural part of the prophylaxis against infections in the urogenital region is enhanced personal hygiene. However, it can be unsuitable to wash genitals and lower abdomen with an excessively strong soap and bactericidal substances, and consequently it may be difficult for an individual to reduce the risk of infection to a sufficient level with the aid of conventional means.

Traditionally, the aforesaid problems are addressed by treating an infection with conventional antibiotics. However, frequent treatment with antibiotics leads to the development of resistant bacteria strains, which can make continued treatment of new infections very difficult. A further problem with antibiotic treatment is that many individuals are hypersensitive to antibiotics. Yet another problem with antibiotic treatment is that the microbiological flora in and around the anus is complex and relatively undefined. It is therefore difficult to propose a prophylactic treatment for reducing the risk of the occurrence of infections that can be caused by microorganisms from the intestines.

Hitherto, the only available method for reducing the risk of infection in the urogenital region has been treatment with antibiotics. However, since the use of antibiotics for a prophylactic purpose is unsuitable for several reasons, there is a serious need for an alternative solution to the problem by generating and maintaining a desired microbiological flora in the urogenital region.

### DESCRIPTION OF THE BACKGROUND ART

As earlier mentioned, a traditional method of dealing with the aforesaid problems is to treat the patient with conventional antibiotics. Various alternative methods of dealing with the aforedescribed problems have been proposed. The use of bacteria as so-called probiotics as an alternative to antibiotics is part of this new methodology.

It is known that certain lactic acid bacteria can have an inhibiting effect on other microorganisms. Application of such lactic acid bacteria has been found to prevent the occurrence of infections on both skin and mucus membrane.

Medical use of selected strains of lactobacteria is described in Canadian Patent Specification CA 1298556 (Bruce, Reid), where, among other things, whole cells or fragments of cells of Lactobacillus are used to treat or to prevent the occurrence of urinal tract infections and intestinal infections.

International Patent Application WO 93/09793 (Reid) describes the use of lactobacteria and skim milk preparations to prevent the occurrence of urogenital infections.

Both CA 1298556 (Bruce, Reid) and WO 93/09793 (Reid) describe the ability of the microorganisms to fasten to the mucus membrane walls, e.g. to the uroepithelium cells or vaginal epithelium cells as an important component for the function of the treatment. However, neither CA 1298556 (Bruce, Reid) nor WO 93/09793 (Reid) describes how the substance concerned shall be applied to the user.

International Patent Application WO 92/13577 (Kvanta) teaches a tampon or sanitary napkin that has been impregnated with a culture of lactic acid producing bacteria, preferably of the genus Pediococcus, that has been isolated from healthy individuals. The tampon or sanitary napkin is intended for prophylactic treatment of urogenital infections.

WO 92/13577 (Kvanta) teaches that applied microorganisms of Lactobacillus are attenuated when technically handled, and the method described relates to treatment and prophylaxis around the actual urethra orifice.

The above cited documents are all solely directed to administration of lactic acid bacteria to the skin. However, none of the documents discloses anything about providing absorbent articles comprising lactic acid bacteria, which articles can be stored for a long time and still contain a sufficient amount of viable and transferable bacteria. It is absolutely necessary that consumer products such as absorbent articles can be stored for a long time and under non-ideal conditions without risking that the quality of the articles is impaired. Consequently, there is a need for absorbent articles which articles are specially adopted for long-time storage under unfavourable conditions.

### SUMMARY OF THE INVENTION

It has now turned out that the above mentioned problems can be overcome, and a high quality can be ensured, by applying a suspension of lactic acid bacteria to an absorbent product, whereafter the absorbent product is dried to a moisture content of less than 10 %, preferably less than 5 %, and most preferably less that 1 %, calculated as percentage of weight of the absorbent core in the product. The absorbent product can be a diaper, sanitary napkin, panty liner, incontinence guard or like article. As already mentioned, it contains lactic acid bacteria and the article is intended to be carried in contact with the user's skin in the perineum area, wherein lactic acid bacteria are arranged to be transferred to the user's skin and, when applicable, to the mucus membrane in the perineum area, to form a microbiological barrier that impairs the conditions for spreading and establishment of undesirable stains of microorganisms in said perineum area.

### BRIEF DESCRIPTION OF THE DRAWINGS

The invention will now be described in greater depth with reference to the accompanying drawings.

Fig. 1 shows the results obtained with interference tests between selected lactic acid bacteria and undesirable microorganisms.

Fig. 2 illustrates schematically the construction of an inventive test product in the form of an insert.

### DETAILED DESCRIPTION OF THE INVENTION

Thus, an inventive absorbent article is intended to transfer lactic acid bacteria to the wearer's perineum and thereby produce a microbiological flora that impairs in the perineum the living conditions for undesirable strains of microorganisms. Undesirable microorganisms are therewith prevented from spreading from the wearer's anus to the urogenital organs.

The lactic acid bacteria, or a preparation that contains lactic acid bacteria, is applied to an absorbent article such as a diaper, sanitary napkin, panty liner, incontinence guard and a like article.

Generally, the lactic acid bacteria are applied to the article by adding a suspension of said bacteria to the surface of the article that is intended to face the wearer. Preferably, this suspension contains skim milk. It is advantageous that the suspension is concentrated in order to reduce the total volume of the suspension, but on the other hand a highly concentrated suspension may lead to clogging problems in pipes and nozzles. A suitable concentration is within the range from 1 x 10⁹ to 1 x 10¹² cfu/ml (colony-forming units per ml), and preferably within the range from 1 x 10¹⁰ to 1 x 10¹¹ cfu/ml. This suspension can be added by spraying or pouring it onto the article. The aqueous part of the suspension is immediately absorbed into absorbing layers in the lower part of the article leaving the bacteria in the upper part of the article that is closest to the wearer in a substantially dry state. Despite this absorption, it has turned out to be essential to carry out a drying step in order to ensure that the moisture content of the finished article is less than 10 %, preferably less than 5 %, and most preferably less than 1 %, calculated as percentage of the weight of the absorbent core in the product.

The lactic acid bacteria will preferably have an inhibiting effect on the growth of undesirable microorganisms in the perineum.

Lactic acid bacteria that are suitable for use in accordance with the invention include one or more strains of the genera Lactobacillus, Lactococcus or Pediococcus. However, the lactic acid bacteria will preferably consist of one or more strains from the Lactobacillus genus.

The lactic acid bacteria can also be combined with other components, such as a pH-lowering substance or an auxiliary substance that will facilitate survival of the lactic acid bacteria. Powdered skimmed milk is one example of such an auxiliary substance.

An inventive absorbent article can be combined advantageously with the local administration of lactic acid bacteria in the vagina with the aid of a capsule, vagitorium or some like means. The inventive substance can also be used advantageously in direct conjunction with antibiotic treatment, for instance the treatment of a urogenital infection.

The invention can be applied with all types of absorbent articles that are intended to be worn in contact with the user's perineum. The lactic acid bacteria may therewith be included as a component in the absorbent body of the absorbent article, or applied in or on the liquid-permeable casing sheet of the article, or in or on a liquid transport layer between the liquid-permeable casing sheet and the absorbent body, or may be applied on a separate carrier, such as a tissue layer or the like. The absorbent article may be a diaper, a sanitary napkin, an incontinence guard, or a panty liner. Such articles normally include an absorbent body or pad enclosed in a casing, wherein the casing suitably includes a liquid-permeable outer sheet over the surface that is intended to lie proximal to the wearer in use. An advantage is afforded when a liquid barrier layer, for instance in the form of plastic film, is arranged adjacent the opposite surface which lies distal from the wearer in use.

When the lactic acid bacteria are disposed inwardly of the article casing, it is essential with respect to the invention that the bacteria are able to pass through the casing at that surface of the article which is intended to lie against the wearer's skin in the perineum.

It has been found to be advantageous when the number of lactic acid bacteria in the absorbent article is between 10⁴ cfu and 10¹¹ cfu, and preferably between 10⁶ cfu and 10¹⁰ cfu.

Alternatively, the absorbent article may have the form of an insert that includes means for its attachment to the liquid-permeable outer sheet of a conventional absorbent article. One advantage with the use of such an insert is that it avoids the need to produce products provided with lactic acid bacteria in a large number of different sizes and models.

As already mentioned, the object of the present invention is to provide an absorbent article of the aforementioned kind that will reduce spreading of bacteria from the anus to the urogenital organs with the aid of microbiological antagonism. This object has been achieved in accordance with the invention with an absorbent article that includes lactic acid bacteria. The absorbent article is intended to be used regularly and in contact with the wearer's skin in the perineum area. By regular use may, in this case, mean daily use or almost daily use of the inventive article. The lactic acid bacteria are therewith applied to the user's perineum area so as to produce and maintain in this area a microbiological flora that impairs the living conditions for undesirable microorganisms in said perineum area, and therewith prevent spreading of these microorganisms from the anus to the urogenital organs.

Examples of species that are associated with urogenital infections are Escherichia coli, Enterobacter, Klebsiella, Pseudomonas, Proteus. Staphylococcus saprophyticus, Staphylococcus epidermidis, group B streptococci, Enterococci, Candida sp., Clamydia sp., Gardnerella vaginalis, Mobiluncus and Bacteroides sp.

As before mentioned, the invention is based on microbiological antagonism. Microbiological antagonism implies that one microorganism or combinations of microorganisms will inhibit other microorganisms. An antagonistic strain shall exhibit growth inhibiting effects with conventional interference techniques on several of the aforesaid undesirable microorganisms. Other important requirements of a suitable antagonistic microorganism is its ability to survive during storage and its growth ability or ability to maintain its activity in a product during use.

Antagonistic microorganisms may be naturally occurring organisms that are non-toxic and that do not exert any negative biological effect on human beings, in the form of infection or skin changes. However, antagonistic microorganisms can also be produced by biotechnical processes.

Certain strains of lactic acid bacteria have a powerful inhibiting effect on undesirable bacteria strains in the user's perineum. This inhibitory effect has its foundation in the fact that lactic acid bacteria possess a number of antagonistic properties that act through different mechanisms, such as by lowering pH, e.g. by producing lactic acid, competition for available nutrients, reduction of the redox-potential, production of hydrogen peroxide, production of specific inhibiting substances or components, such as enzymes, toxins or bacteriocines and competition for available binding sites. The antagonistic effect can be elevated still further in some instances, by adding an additional pH-lowering substance.

The growth of undesirable microorganisms present in the perineum of the user can be inhibited by adding to the inventive product lactic acid bacteria that exhibit antagonistic properties against said undesirable microorganisms. At least some microorganisms of undesired species can also be killed in this way. The microorganisms added to the product must be added in such quantities and have such activities as to achieve the effect desired. This effect is normally achieved when the number of antagonistic microorganisms per product is greater than 10⁶ cfu, preferably 10⁸ cfu and more preferably 10⁹ cfu.

One advantage afforded by the use of antagonistic microorganisms is that one avoids an undesired selection pressure on the microenvironment, such as favouring of potentially pathogenic microorganisms and therewith the risk of developing pathogenic strains that are resistant to antibiotics and chemopharmaceuticals. Since the antimicrobial system is based on a natural biological process, the risk of ecological and toxic environmental disturbances is reduced.

The inventive product can include a carrier in the form of, e.g., a typical panty liner with or without a liquid-impervious backing sheet and including an absorbent layer which contains 100-200 g/m² cellulose pulp mixed with 0-10% superabsorbent powder. That side of the product which is intended to lie proximal to the wearer in use includes lactic acid bacteria in a concentration that will preferably be in the order of 10⁴-10¹¹, preferably 10⁶-10¹⁰ cfu per product. An inventive product will preferably include a suitable substance that will assist in the survival of the microorganisms. One such aid may, e.g., be powdered skim milk. Regardless of the general form of the product, an inventive product may also include a suitable pH-lowering substance for further enhancing the antagonistic effect.

### EXAMPLES

The following examples are intended to further illustrate the effect of an inventive product

### Example 1

Bacterial antagonism was studied with the aid of tests carried out in accordance with the agar overlay method. The method is based on the diffusion of the growth inhibiting substance produced by the lactic acid bacteria through an agar layer and their inhibition of the growth of the test organisms.

Lactic acid bacteria, three strains of Lactobacillus designated LB13, LB14 and LB16 respectively, and five strains of Lactococcus designated L3, L4, L5, L9 and L26 respectively, were cultivated to an overnight culture in nutrient broth from Merck. Lactococcus were cultivated in M17 and Lactobacillus in MRS. Agar (2%) of M17 and MRS (25 ml) respectively was mixed with 1.0 ml of respective bacteria and moulded in a Petri dish. The agar plates were incubated overnight at 37°C. The plates with MRS were incubated in a CO₂ atmosphere. Reference plates were prepared in a corresponding manner, but without lactic acid bacteria. A fresh layer containing 25 ml agar was cast on top of the existing layer in the Petri dishes and allowed to solidify.

The test organisms in the form of gram-negative bacteria of respective Escherichia coli, Klebsiella spp and Proteus spp and 100, 91 and 50 strains respectively were cultivated in a broth, and a dilution corresponding to 10⁷ cfu/ml was prepared in Bertani trays. The test bacteria were then stamped on the new agar layer with the aid of a Steers Steel Pin Replicator (Steers E et al, J. Antibiot Chemother (1979,9,307). The plates were incubated at 37°C for twenty-four hours. Subsequent to incubation, the plates were read and compared with the reference plates. When reading the plates, "growth", "inhibition" or "zero growth" were registered for respective test organisms. The pH of all agar layers was measured, and those plates with a pH below 5.0 were re-tested with pH-adjusted agar, i.e. with agar to which a small quantity of glucose was added to counteract pH reduction. The percentile proportion of the total number of test organisms that had been inhibited or gave zero growth was calculated.

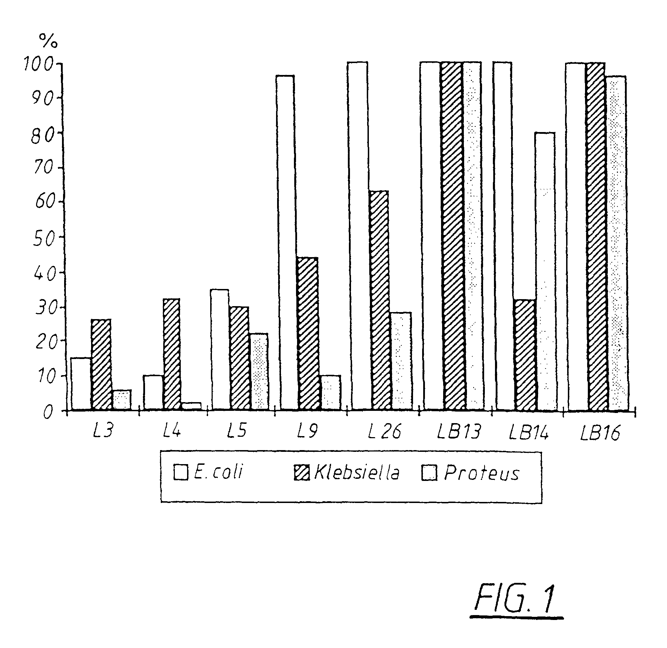

The diagram in Fig. 1 shows the percentage of the different strains of Escherichia coli, Klebsiella and Proteus that were inhibited by the presence of the selected strains of genera Lactobacillus and Lactococcus. It is evident that the Lactobacillus strains LB13 and LB16 have an inhibitory effect on practically all pathogenic strains. The Lactobacillus strain designated LB14 inhibited the growth of all E.coli strains, about 80% of the Proteus strains and slightly more than 30% of the Klebsiella strains. All of the Lactococci and Lactobacilli used had a growth inhibiting effect on some of the pathogenic strains. Of the Lactococci strains, inhibition of the highest percentage of pathogens was obtained with L26 and L9, both of which had an inhibitory effect on practically all E. coli strains and on a large percentage of the Klebsiella strains.

### Example 2

The following tests were carried out with the intention of studying the transfer of lactic acid bacteria to the perineum of persons wearing panty liners. All test persons were females between 3-60 years of age. In some cases, the test was carried out between the menstruation periods of the test persons concerned. Test products were produced from conventional panty liners that included a liquid-permeable casing sheet, a liquid-impermeable back sheet, and an absorbent layer of chemical cellulose pulp, 100-200 g/m², sandwiched therebetween. A suspension of selected lactic acid bacteria was sprayed on the absorbent side of the test products, in a concentration of 10⁹ cfu per product.

The presence of lactic acid bacteria in the perineum of the test persons was determined with the aid of a so-called swab test. In this case, bacteria were collected by dipping a sterile stick provided with a cotton-wool top into a sterile saline solution and stroking the top of the stick over a defined area of the skin. The presence of lactic acid bacteria in the perineum of twenty test persons was determined, measured, in this way, to obtain a so-called 0-sample (background sample). The test persons then wore the panty liner for five hours over a morning period. The panty liners were then removed and the presence of lactic acid bacteria again measured, immediately after removing the panty liners. This test was designated Sample 1. A further test, designated Sample 2, was run after a further four-five hours. The type of lactic acid bacteria was identified on each sampling occasion, in order to ascertain that none of the test persons was a natural carrier of the selected type of lactic acid bacteria added to the test products. The identification method used was API (API-system, La Balme les Grottes, 38390 Montalieu, Vercieu, France).

The results obtained with these measuring processes are defined in Table 1.

**Table 1**

| | | Presence of LAB* in the Perineum (cfu) | | |
|---|---|---|---|---|
| TP | Flora | 0-sample | Sample 1 | Sample 2 |
| 1 | Added LAB | 0 | 0 | 0 |
| | Own LAB | 0 | 0 | 0 |
| | | | | |
| 2 | Added LAB | 0 | 4.0x10¹ | 0 |
| | Own LAB | 0 | 0 | 0 |
| | | | | |
| 3 | Added LAB | 0 | 7.2x10³ | 1.6x10² |
| | Own LAB | 0 | 0 | 0 |
| | | | | |
| 4 | Added LAB | 0 | 0 | 0 |
| | Own LAB | 0 | 0 | 0 |
| | | | | |
| 5 | Added LAB | 0 | 1.1x10² | 5.0x10¹ |
| | Own LAB | 0 | 0 | 0 |
| | | | | |
| 6 | Added LAB | 0 | 5.0x10¹ | 3.0x10¹ |
| | Own LAB | + | + | 0 |
| | | | | |
| 7 | Added LAB | 0 | 3.2x10² | 1.3x10³ |
| | Own LAB | + | + | + |
| | | | | |
| 8 | Added LAB | 0 | 2.7x10³ | 6.0x10² |
| | Own LAB | 0 | 0 | 0 |
| | | | | |
| 9 | Added LAB | 0 | 3.2x10³ | 1.0x10¹ |
| | Own LAB | + | 0 | + |
| | | | | |
| | | | | |
| 10 | Added LAB | 0 | 7.6x10² | 7.0x10¹ |
| | Own LAB | + | + | + |
| 11 | Added LAB | 0 | 8.4x10³ | 7.0x10¹ |
| | Own LAB | + | + | + |
| | | | | |
| 12 | Added LAB | 0 | 3.5x10³ | 8.0x10¹ |
| | Own LAB | 0 | 0 | 0 |
| 13 | Added LAB | 0 | 2.4x10² | 3.0x10² |
| | Own LAB | + | + | + |
| | | | | |
| 14 | Added LAB | 0 | 0 | 0 |
| | Own LAB | + | + | + |
| | | | | |
| 15 | Added LAB | 0 | 2.1x10² | 8.0x10¹ |
| | Own LAB | + | + | + |
| | | | | |
| 16 | Added LAB | 0 | 1.6x10⁴ | 4.0x10³ |
| | Own LAB | 0 | 0 | 0 |
| | | | | |
| 17 | Added LAB | 0 | 6.0x10² | 1.5x10² |
| | Own LAB | + | + | + |
| | | | | |
| 18 | Added LAB | 0 | 9.3x10² | 1.0x10¹ |
| | Own LAB | 0 | 0 | 0 |
| | | | | |
| 19 | Added LAB | 0 | 4.0x10¹ | 1.0x10¹ |
| | Own LAB | 0 | 0 | 0 |
| 20 | Added LAB | 0 | 7.2x10³ | 7.0x10¹ |
| | Own LAB | 0 | 0 | 0 |
| | | | | |
| Number of TP with added LAB | | 0 | 17 | 16 |

| | | | | |
|---|---|---|---|---|
| *LAB = lactic acid bacteria | | | | |
| 0 = no lactic acid bacteria were found on the test persons | | | | |
| + = presence of own LAB on the test persons | | | | |

As evident from Table 1, the selected lactic acid bacteria added to the test products were activated when the test products were worn by the test persons, and that the selected lactic acid bacteria were transferred to the perineum area of said persons and found to be present in said area even for a relatively long period of time after having removed the test products.

### Example 3

A clinical study was carried out with the aim of illustrating the effect of adding lactic acid bacteria to the uro-perineal flora of children suffering from myelomeningocele. The study was blind, randomised and with cross-over design. Twenty-three children aged from two to seventeen participated in the study. All children were diaper wearers. Test products in the form of inserts with and without lactic acid bacteria were placed nearest the skin in the standard diaper.

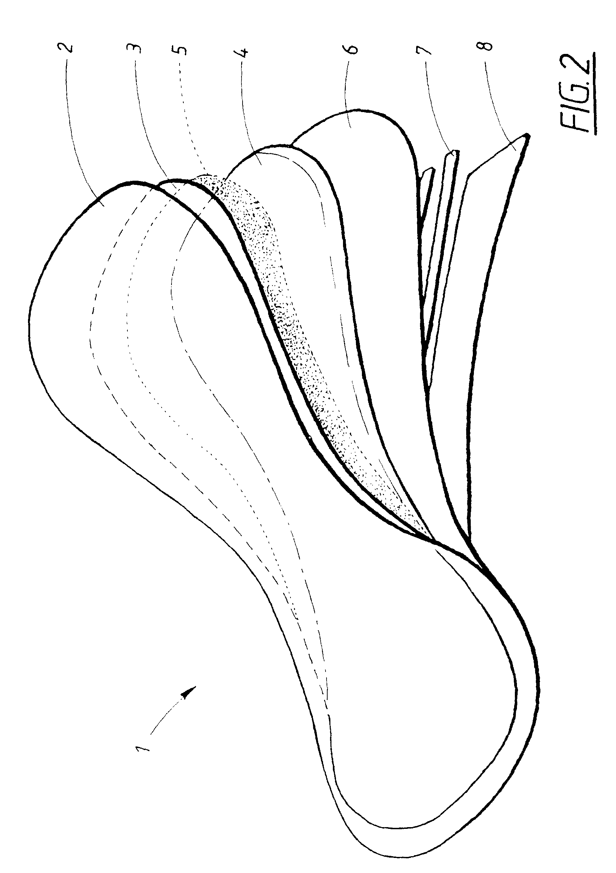

The construction of the insert 1 used is shown in Fig. 2. Arranged uppermost on the insert 1, i.e. nearest the wearer's skin, is a liquid-permeable casing sheet 2 made of nonwoven material. A thin tissue layer 3 is disposed immediately adjacent to and inwardly of the casing sheet 2. The insert 1 also includes an absorbent pad 4 consisting of a mixture of cellulose fluff pulp and superabsorbent material. Placed on the surface of the absorbent pad 4 proximal to the tissue layer 3 is a layer of freeze-dried milk powder 5 with or without an admixture of selected, freeze-dried lactic acid bacteria in a concentration of 10⁹ cfu per insert. The tissue layer 3, the absorbent pad 4 and the powdered milk 5 were enclosed between the liquid-permeable casing sheet 2 and a similar liquid-permeable backing sheet 6 of nonwoven material.

Two glue strings 7 were provided on the bottom surface 6 intended to lie proximal to the diaper in use, to enable the insert 1 to be fastened to the diapers worn by the test children. The glue strings 7 were protected by release paper 8, prior to placing the insert 1 in respective diapers.

The absorbent pad 4 was formed as an absorbent layer of 150 g/m² chemithermomechanical pulp admixed with 10% superabsorbent powder. The freeze-dried milk powder, in the present case admixed with freeze-dried lactic acid bacteria, was sprinkled on the surface of the tissue layer 3 facing the absorbent pad 4. The tissue layer 3 had a weight per unit area of about 20 g/m² and was placed on top of the milk powder. The casing sheet 2 and the backing sheet 6 consisted of a nonwoven material having a weight per unit area of about 17 g/m² and were laminated in both sides by the absorbent unit 3-5. The thus produced insert 1 had an hourglass configuration with a largest width of about 9 cm, a smallest width of about 6.5 cm, and a length of about 24 cm.

The tests were continued over periods of 6 + 6 weeks with an intermediate period of three weeks. A urine culture was made prior to and during respective test periods (at two-week intervals) and a quantitative determination was made from the perineum and the urethra orifice. The number of bacteria obtained were transferred to a measurement scale between 0 and 5. where 0 denotes cfu <10², 1 denotes cfu between 10² and 10³, and so on.
The results obtained are set forth in Table 2.

**Table 2**

| | | Insert without LAB* | | Insert with LAB* | | |
|---|---|---|---|---|---|---|
| Bacteria group | Local | M | SD | M | SD | Signicance |
| Potential | Perineum | 1.93 | 0.84 | 1.52 | 0.89 | Yes |
| Urinal | Urethra | 1.62 | 1.12 | 1.29 | 0.95 | No |
| Tract Pathogens | Urine | 1.93 | 1.72 | 1.43 | 1.39 | Yes |

| | | | | | | |
|---|---|---|---|---|---|---|
| *LAB = lactic acid bacteria | | | | | | |
| M = mean value | | | | | | |
| SD = standard deviation | | | | | | |

It is evident from Table 2 that the insert containing selected lactic acid bacteria provides a statistically significant (p < 0.05) reduction in the number of potential urinal tract pathogens (PUP) in the perineum and in the urine.

### Example 4

The following tests were carried out with the intention of studying the survival of lactic acid bacteria in absorbent articles according to the invention. Test products were produced from conventional panty liners that included a liquid-permeable casing sheet, a liquid-impermeable back sheet, and an absorbent layer of chemical cellulose pulp, 100-200 g/m², sandwiched therebetween. About 150 µl of a suspension of selected lactic acid bacteria in saline skim milk sprayed on the absorbent side of the test products, in a concentration of about 9.0 x 10⁷ cfu per product. Some test products were dried to a moisture content of less than 10 % (wt.) before they were packed in plastic packages, whereas other products were packed without any preceding drying step. The amount of living lactic acid bacteria was determined after 14, 28, 40 and 53 days, respectively using standard methods. The results of these tests are disclosed in Table 3 below:

**Table 3**

| | dried panty liner | | panty liner that has not been dried | |
|---|---|---|---|---|
| Day | cfu | cfu | cfu | cfu |
| 0 | 9.0 x 10⁷ | 7.0 x 10⁷ | 9.0 x 10⁷ | 7.0 x 10⁷ |
| 14 | 7.5 x 10⁶ | 6.1 x 10⁶ | 3.6 x 10⁵ | 8.0 x 10⁵ |
| 28 | 7.2 x 10⁷ | 2.6 x 10⁷ | 2.1 x 10⁴ | 1.0 x 10³ |
| 40 | 8.9 x 10⁷ | 3.2 x 10⁷ | 1.4 x 10⁴ | 1.0 x 10³ |
| 53 | 7.0 x 10⁷ | 3.0 x 10⁷ | 1.1 x 10⁴ | 1.0 x 10³ |

As is evident from table 3 above, the amount of viable lactic acid bacteria does not decrease during the test period. However, the amount of viable lactic acid bacteria decreases considerably if no drying step is carried out before packaging.

## Claims

1. An absorbent product, such as a diaper, sanitary napkin, panty liner, incontinence guard, an insert or like article, which contains lactic acid bacteria and is intended to be carried in contact with the user's skin in the perineum area, wherein lactic acid bacteria are arranged to be transferred to the user's skin and, when applicable, to the mucus membrane in the perineum area, to form a microbiological barrier that impairs the conditions for spreading and establishment of undesirable stains of microorganisms in said perineum area, **characterised in that** said absorbent product has been produced by applying a suspension of said lactic acid bacteria whereafter the absorbent product is dried to a moisture content of less than 10 %, preferably less than 5 % and most preferably less than 1 %, calculated as percentage of the weight of the absorbent core in the product.

2. A product according to claim 1, wherein the lactic acid bacteria exhibit an inhibiting effect against undesirable microorganisms in the user's perineum.

3. A product according to any one of the preceding Claims, wherein the lactic acid bacteria include one or more strains from the genera Lactobacillus, Lactococcus or Pediococcus.

4. A product according to Claim 3, wherein the lactic acid bacteria consist of one or more strains from the genus Lactobacillus.

5. A product according to any one of the preceding Claims, wherein the product includes a pH-lowering substance.

6. A product according to any one of the preceding Claims, wherein the product includes an auxiliary substance for facilitating survival of the lactic acid bacteria.

7. A product according to Claim 6, wherein said auxiliary substance is powdered skim milk.

8. An absorbent article according anyone of Claims 1-7, wherein lactic acid bacteria are disposed in or on a liquid-permeable casing sheet (2) included in said article.

9. An absorbent article according to anyone of claims 1-7, wherein lactic acid bacteria are located directly below the liquid-permeable casing sheet (2).

10. An absorbent article according to Claim 8 or 9, wherein lactic acid bacteria are disposed in or on an absorbent pad (4) included in said article.

11. An absorbent article according to any one of Claims 1-10, wherein the article contains lactic acid bacteria in numbers from between 10⁴ and 10¹¹ cfu, preferably between 10⁶ and 10¹⁰ cfu.

## Patentansprüche

1. Absorbierendes Produkt, wie eine Windel, Damenbinde, Slipeinlage, ein Inkontinenzschutz, ein Gegenstand zum Einführen oder Einlegen oder vergleichbarer Gegenstand, welcher Milchsäurebakterien enthält und in Kontakt mit der Haut des Verwenders im Perineumbereich getragen werden soll, wobei die Milchsäurebakterien für eine Übertragung auf die Haut des Verwenders und, soweit anwendbar, auf die Mukusmembran im Perineumbereich angeordnet sind, zwecks Bildung einer mikrobiologischen Barriere, welche die Bedingungen zum Verbreiten und Festsetzen unerwünschter Stämme an Mikroorganismen in dem besagten Perineumbereich verschlechtert, **dadurch gekennzeichnet, daß** das absorbierende Produkt durch Aufbringen einer Suspension der Milchsäurebakterien hergestellt wurde, wonach man das absorbierende Produkt auf einen Feuchtigkeitsgehalt von weniger als 10 %, vorzugsweise weniger als 5 % und am stärksten bevorzugt weniger als 1 %, berechnet als Gewichtsprozent des absorbierenden Kerns im Produkt, trocknet.

2. Produkt gemäß Anspruch 1, worin die Milchsäurebakterien eine inhibierende Wirkung gegenüber unerwünschten Mikroorganismen im Perineum des Verwenders aufweisen.

3. Produkt gemäß einem der vorangehenden Ansprüche, worin die Milchsäurebakterien einen oder mehr Stämme der Gattung Lactobacillus, Lactococcus oder Pediococcus enthalten.

4. Produkt gemäß Anspruch 3, worin die Milchsäurebakterien aus einem oder mehr Stämmen der Gattung Lactobacillus bestehen.

5. Produkt gemäß einem der vorangehenden Ansprüche, worin das Produkt eine pH-verringernde Substanz enthält.

6. Produkt gemäß einem der vorangehenden Ansprüche, worin das Produkt eine Hilfssubstanz zum Erleichtern des Überlebens der Milchsäurebakterien enthält.

7. Produkt gemäß Anspruch 6, worin die Hilfssubstanz pulverisierte Magermilch ist.

8. Absorbierender Gegenstand gemäß einem der Ansprüche 1 bis 7, worin die Milchsäurebakterien in oder auf einer flüssigkeitsdurchlässigen Hüllage (2) angeordnet sind, die von dem Gegenstand umfaßt wird.

9. Absorbierender Gegenstand gemäß einem der Ansprüche 1 bis 7, worin die Milchsäurebakterien direkt unterhalb der flüssigkeitsdurchlässigen Hüllage (2) lokalisiert sind.

10. Absorbierender Gegenstand gemäß Anspruch 8 oder 9, worin die Milchsäurebakterien in oder auf einem absorbierenden Polster (4) angeordnet sind, das von dem Gegenstand umfaßt wird.

11. Absorbierender Gegenstand gemäß einem der Ansprüche 1 bis 10, worin der Gegenstand Milchsäurebakterien in einer Zahl von zwischen 10⁴ und 10¹¹ cfu, vorzugsweise zwischen 10⁶ und 10¹⁰ cfu, enthält.

## Revendications

1. Produit absorbant, tel qu'une couche-culotte, une serviette hygiénique, un protège-culotte, une protection contre l'incontinence, une pièce d'insertion, ou un article analogue, qui contient des bactéries à acide lactique et qui est conçu pour être porté en contact avec la peau de la personne dans la zone du périnée, dans lequel les bactéries à acide lactique sont agencées pour être transférées à la peau de l'utilisateur, et, lorsque cela est applicable, à la membrane muqueuse dans la zone du périnée, de façon à former une barrière microbiologique qui gène les conditions de dispersion et d'établissement de taches indésirables de micro-organismes dans ladite zone du périnée, **caractérisé en ce que** ledit produit absorbant a été produit en appliquant une suspension desdites bactéries à acide lactique, après quoi le produit absorbant est séché sous une proportion d'humidité inférieure à 10%, de préférence inférieure à 5%, et, de la façon la plus préférable, inférieure à 1%, calculée en pourcentage du poids du coeur absorbant dans le produit.

2. Produit selon la revendication 1, dans lequel les bactéries à acide lactique présentent un effet inhibant contre les micro-organismes indésirables dans le périnée de la personne.

3. Produit selon l'une quelconque des revendications précédentes, dans lequel les bactéries à acide lactique comprennent une ou plusieurs souches des genres Lactobacillus, Lactococcus ou Pédiococcus.

4. Produit selon la revendication 3, dans lequel les bactéries à acide lactique comprennent une ou plusieurs souches du genre Lactobacillus.

5. Produit selon l'une quelconque des revendications précédentes, dans lequel le produit comprend une substance d'abaissement du pH.

6. Produit selon l'une quelconque des revendications précédentes, dans lequel le produit comprend une substance auxiliaire pour faciliter la survie des bactéries à acide lactique.

7. Produit selon la revendication 6, dans lequel ladite substance auxiliaire est de la crème de lait en poudre.

8. Article absorbant selon l'une quelconque des revendications 1 à 7, dans lequel les bactéries à acide lactique sont disposées dans ou sur une feuille d'enveloppe perméable aux liquides (2) incluse dans ledit article.

9. Article absorbant selon l'une quelconque des revendications 1 à 7, dans lequel les bactéries à acide lactique sont disposées directement en dessous de la feuille d'enveloppe perméable aux liquides (2).

10. Article absorbant selon la revendication 8 ou 9, dans lequel les bactéries à acide lactique sont disposées dans ou sur un tampon absorbant (4) inclus dans ledit article.

11. Article absorbant selon l'une quelconque des revendications 1 à 10, dans lequel l'article contient des bactéries à acide lactique sous des nombres compris entre 10⁴ et 10¹¹ unités par pied cube (1 pied cube = 92.903 cm³), et, de préférence, entre 10⁶ et 10¹⁰ unités par pied cube.
